(19) Europäisches Patentamt
European Patent Office
Office européen des brevets

(11) Veröffentlichungsnummer: **0 132 741**
**B1**

(12) # EUROPÄISCHE PATENTSCHRIFT

(45) Veröffentlichungstag der Patentschrift: **11.03.87**

(21) Anmeldenummer: **84108332.2**

(22) Anmeldetag: **16.07.84**

(51) Int. Cl.⁴: **C 07 C 50/14,** C 07 C 50/28, C 07 C 50/22, C 07 C 50/34, C 07 C 50/20

(54) **Verfahren zur Herstellung von Verbindungen der Vitamin K-Reihe und von Ubichinonen, sowie neue Zwischenprodukte.**

(30) Priorität: **28.07.83 CH 4143/83**
**16.05.84 CH 2398/84**

(43) Veröffentlichungstag der Anmeldung: **13.02.85 Patentblatt 85/7**

(45) Bekanntmachung des Hinweises auf die Patenterteilung: **11.03.87 Patentblatt 87/11**

(84) Benannte Vertragsstaaten: **AT BE CH DE FR GB IT LI NL**

(56) Entgegenhaltungen:
**AT - B - 235 827**
**DE - A - 2 416 035**
**DE - A - 2 642 670**
**DE - B - 1 054 085**
**DE - C - 905 490**
**US - A - 4 374 775**

**HOUBEN-WEYL "Methoden der organischen Chemie", 4. Auflage, Band V/1c: "Kohlenwasserstoffe", Teil 3, 1970, Georg Thieme Verlag, Stuttgart, Seite 1028, Tabelle 10**

(73) Patentinhaber: **F. HOFFMANN-LA ROCHE & CO. Aktiengesellschaft, CH-4002 Basel (CH)**

(72) Erfinder: **Rüttimann, August, Dr., General Guisan-Strasse 41, CH-4144 Arlesheim (CH)**
Erfinder: **Büchi, George H., Prof. Dr., Pinkham Notch Road, Jackson, NH 03846 (US)**

(74) Vertreter: **Cottong, Norbert A. et al, Grenzacherstrasse 124 Postfach 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur Herstellung von Chinonderivaten, insbesondere von Verbindungen der Vitamin K-Reihe und von Ubichinonen. Die Erfindung betrifft ebenfalls neue Ausgangs- und Zwischenprodukte in diesem Verfahren.

Die bisher bekannten Verfahren zur Herstellung von derartigen Verbindungen verlaufen in der Regel ausgehend von Hydrochinonen oder monoacylierten Hydrochinonen und sind daher technisch nicht zufriedenstellend, da relativ viele Reaktionsstufen durchlaufen werden müssen. Technisch praktikable Verfahren ausgehend von z.B. Menadion selbst oder einem leicht zugänglichen Derivat hiervon sind bisher nicht bekannt. Diese Lücke sind nun mittels des erfindungsgemässen Verfahrens geschlossen, da dieses die Herstellung von Vitaminen der K-Reihe sowie von Ubichinonen in einer oder zwei Stufen, ausgehend von leicht zugänglichen Ausgangsmaterialien, erlaubt. Das erfindungsgemässe Verfahren ist zudem von besonderem Interesse, da es, im Gegensatz zu vielen vorbekannten Verfahren, unter praktisch vollständiger Retention der Konfiguration der Doppelbindung(en) in der Seitenkette abläuft.

Das erfindungsgemässe Verfahren ist dadurch gekennzeichnet, dass man eine Verbindung der allgemeinen Formel

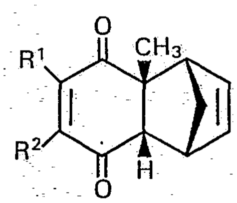

I

worin $R^1$ und $R^2$ Methoxygruppen oder zusammen die Gruppe -CH=CH-CH=CH- darstellen, mit einer Verbindung der allgemeinen Formel

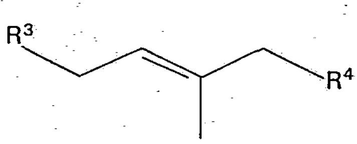

II

worin $R^3$ eine Abgangsgruppe bedeutet und $R^4$ den 3,7,11-Trimethyldodecanrest oder einen Rest der Formel

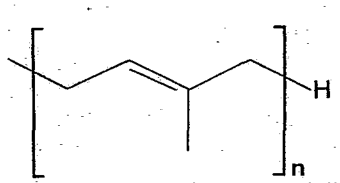

III

darstellt, worin n eine Zahl 0-12 bedeutet, umsetzt und gewünschtenfalls eine so erhaltene Verbindung der allgemeinen Formel

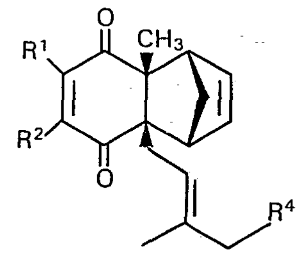

IV

worin $R^1$, $R^2$ und $R^4$ die obige Bedeutung haben, in eine Verbindung der allgemeinen Formel

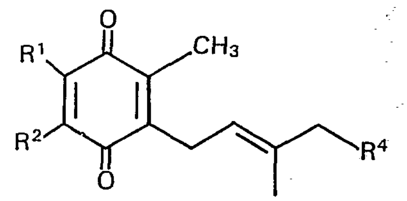

V

worin $R^1$, $R^2$ und $R^4$ die obige Bedeutung haben, überführt.

Der Ausdruck «Abgangsgruppe» bedeutet im Rahmen der vorliegenden Erfindung insbesondere Halogen wie Fluor, Chlor, Brom und Jod, wobei Brom und Chlor bevorzugt sind, oder auch Gruppen wie die Mesylgruppe, die Tosylgruppe, die Acetatgruppe und dergleichen. Weiterhin bedeutet das Zeichen «◀», dass sich der entsprechende Rest oberhalb der Molekülebene befindet.

Das erfindungsgemässe Verfahren erlaubt sowohl die Herstellung von trans/cis-Gemischen der Verbindungen der Formel V sowie auch der praktisch reinen (E)- oder (Z)-Isomeren und zwar abhängig von der Konfiguration der Ausgangsverbindungen der Formel II. So kann beispielsweise bei Verwendung von Verbindungen der Formel II in reiner (E)-Form, die entsprechende Verbindung der Formel V in praktisch reiner (E)-Form erhalten werden.

Die Umsetzung einer Verbindung der Formel I mit einer Verbindung der Formel II kann in einem unter den Reaktionsbedingungen inerten organischen Lösungsmittel und in Gegenwart einer starken Base erfolgen. Als Lösungsmittel kommen hier in Frage sowohl polare als auch apolare Lösungsmittel. Bevorzugt sind apolare aprotische Lösungsmittel wie z.B. aliphatische oder aromatische Kohlenwasserstoffe wie Hexan, Benzol, Toluol und dergleichen und tert.-Butanol als polares protisches Lösungsmittel. Ebenfalls bevorzugt sind Gemische dieser Lösungsmittel. Als starke Base kommen im Rahmen der vorliegenden Erfindung insbesondere in Frage organische Basen, wie z.B. Amide wie Alkalimetallamide (Li, Na, K) oder Lithiumdialkylamide, Alkoholate wie Alkalimetall-tert.-Butylate oder auch Hydride wie Natrium- oder Kaliumhydrid und dergleichen. Die Reaktion kann ferner bei einer Temperatur von etwa -20°C bis etwa +30°C, vorzugsweise bei etwa -5°C bis etwa +10°C und insbesondere bei etwa 0°C bis +5°C erfolgen.

Die Verbindungen der Formel IV sind neu und ebenfalls Gegenstand der vorliegenden Erfindung.

Die Überführung einer Verbindung der Formel IV in eine Verbindung der Formel V stellt eine Retro-Diels-Alder-Reaktion dar und kann somit in an sich bekannter Weise durchgeführt werden. Das Erhitzen kann in Abwesenheit oder in Gegenwart eines inerten Lösungsmittels erfolgen, beispielsweise bei einer Temperatur von etwa Raumtemperatur bis etwa 200°C, vorzugsweise bei einer Temperatur von etwa 70°C bis etwa 120°C.

Die als Ausgangsmaterial verwendeten Verbindungen der Formel II sind bekannt und können in bekannter Weise hergestellt werden.

Die in dem erfindungsgemässen Verfahren als Ausgangsmaterial verwendeten Verbindungen der Formel I sind neu und ebenfalls Gegenstand der vorliegenden Erfindung. Sie können beispielsweise dadurch hergestellt werden, dass man ein Chinon der allgemeinen Formel

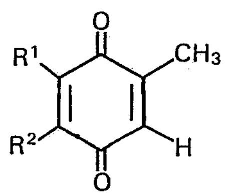

VI

worin $R^1$ und $R^2$ die obige Bedeutung haben, mit Cyplopentadien umsetzt. Diese Umsetzung kann in einem inerten organischen Lösungsmittel und vorzugsweise bei einer Temperatur von etwa 0°C bis etwa 40°C, insbesondere Raumtemperatur durchgeführt werden. Als Lösungsmittel verwendet man vorzugsweise eine organische Säure wie z.B. Essigsäure, Propionsäure und dergleichen.

*Beispiel 1*

In einem Sulfierkolben versehen mit Rührer, Rückflusskühler und Argonbegasung wurden unter Argon 50 ml tert.-Butanol/Toluol (4 : 1) und 1,65 g Kalium (42 mMol) vorgelegt und während 1 Stunde am Rückfluss erhitzt. Hierauf wurde das Gemisch auf 0°C abgekühlt und mit 5 g (21 mMol) 1,4,4aα,9a-Tetrahydro-9aα-methyl-1α,4α-methanoanthrachinon versetzt. Die erhaltene rote Lösung wurde noch 15 Minuten bei 0°C weitergerührt. Anschliessend wurden während ca. 15 Minuten 8,7 g (1,15 Aeq.) trans-Phytylbromid in 10 ml tert.-Butanol/Toluol (4 : 1) zugetropft und das Ganze während 1 Stunde bei 0°C gerührt. Darauf wurden 15 ml 3N HCl zugetropft und die enstandene gelbe Lösung eine halbe Stunde bei Raumtemperatur gerührt. Darauf wurde eine 25%ige Ammoniaklösung zugegeben bis die Lösung orange wurde. Dann wurde die Lösung am Rotationsverdampfer eingeengt und zweimal mit Hexan extrahiert. Die organischen Extrakte wurden mit gesättigter NaCl-Lösung gewaschen, über $Na_2SO_4$ getrocknet, filtriert und eingeengt. Man erhielt 12,5 g eines braunen Öles welches auf einer 400 g $SiO_2$-Säule mit Hexan-Essigsester (19 : 1) chromatographiert wurde. Auf diese Weise erhielt man 10,1 g 1,4, 4a,9a-Tetrahydro-9aα-methyl-4aα-[3,7,11,15-tetramethyl-2-hexadecenyl]-1α,4α-methanoanthrachinon in Form eines gelben Öles.

10,1 g (19,6 mMol) des vorhergehend erhaltenen gelben Öles wurden in ca. 25 ml Toluol gelöst und unter Argon im Dunkeln während 15 Minuten am Rückfluss erhitzt. Dann wurde das Gemisch abgekühlt und am Rotationsverdampfer eingeengt. Man erhielt 9 g eines gelben Öles, welches auf einer 300 g $SiO_2$-Säule mit Hexan-Essigester (19 : 1) chromatographiert wurde. Man erhielt so 8,1 g trans-Vitamin $K_1$ in Form eines gelben Öles.

HPLC: Verhältnis trans/cis = 96,1 : 3,9.

Das als Ausgangsmaterial verwendete 1,4,4aα,9a-Tetrahydro-9aα-methyl-1α,4α-methanoanthrachinon wurde wie folgt hergestellt:

In einem Kolben wurden 103,6 g (0,6 Mol) Menadion in 400 ml Essigsäure vorgelegt. Dann wurden 126 ml 1,3-Cyclopentadien (1,53 Mol) zugesetzt und das Gemisch bei Raumtemperatur gerührt. Nach etwa 2$^1/_2$ Stunden war alles gelöst. Nach 4 Tagen bei Raumtemperatur wurde die Lösung am Rotationsverdampfer bei 50°C eingeengt, der Rückstand bei 0°C aus 280 ml Methanol umkristallisiert, die Kristalle abfiltriert und während 3 Stunden bei 35°C am Wasserstrahlvakuum getrocknet. Man erhielt 121,6 g 1,4,4aα,9a-Tetrahydro-9aα-methyl-1α,4α-methanoanthrachinon mit einem Schmelzpunkt von 95 bis 97°C.

In zum vorhergehenden analoger Weise wurden ausgehend von 1,8 g (7,5 mMol) 1,4,4aα,9a-Tetrahydro-9aα-methyl-1α,4α-methanoanthrachinon und 3,5 g (1,3 Aeq.) cis-Phytylbromid 1,83 g cis-Vitamin $K_1$ in Form eines gelben Öles erhalten. HPLC-Gehalt: 99% cis.

*Beispiel 2*

In einem 200 ml Sulfierkolben versehen mit Rührer, Rückflusskühler und Argonbegasung wurden unter Argon 40 ml tert.-Butanol und 1,65 g (42 mMol) Kalium gegeben und während 1$^1/_2$ Stunden am Rückfluss erhitzt. Hierauf wurde das Gemisch auf Raumtemperatur abgekühlt und mit 10 ml Toluol versetzt. Dann wurde mittels eines Eisbades auf +3°C abgekühlt. Dann wurden 5 g (21 mMol) 1,4aα,9a-Tetrahydro-9aα-methyl-1α,4α-methanoanthrachinon (hergestellt gemäss Beispiel 1) zugesetzt und das Gemisch während 5 Minuten bei +3°C gerührt. Hierauf wurden 9,8 g (27,3 mMol) trans-Phytylbromid in 10 ml tert.-Butanol/Toluol (4 : 1) innert 20 Minuten bei ca. 5°C zugetropft und das Gemisch bei etwa +3°C noch $^1/_2$ bis 1 Stunde nachgerührt. Anschliessend wurden 15 ml (45 mMol) 3N HCl zugesetzt. Dann wurde mit einem warmen Wasserbad auf +25°C erwärmt und 3/4 Stunden bei Raumtemperatur intensiv gerührt. Hierauf wurde 25%ige Ammoniaklösung zugetropft bis zum Umschlag der hellgelben Farbe der Reaktionslösung nach orange. Dann wurde die Reaktionslösung am Rotationsverdampfer bei 25-30°C eingeengt. Der Rückstand wurde in zweimal 300 ml Hexan aufgenommen, einmal mit halbgesättigter NaCl-Lösung und einmal mit gesättigter NaCl-Lösung gewaschen und anschliessend über $Na_2SO_4$ getrocknet. Man erhielt 12,9 eines gelben Öles.

Dieses Öl wurde anschliessend in 25 ml Toluol gelöst und während 15 Minuten unter Argon im Dunkeln am Rückfluss erhitzt. Dann wurde das Gemisch abgekühlt und am Rotationsverdampfer bei 35°C eingeengt. Man erhielt 12,5 g eines gelbroten Öles, welches an 430 g $SiO_2$ mit Hexan/Essigester (19 : 1) chromatographiert wurde. Man erhielt 8,6 g trans-Vitamin $K_1$ in Form eines gelben Öles. HPLC: Verhältnis trans/cis = 96,6 : 3,4.

In zum vorhergehenden analoger Weise wurde bei Verwendung von trans/cis-Phytylbromid trans/cis-Vitamin $K_1$ erhalten. HPLC: Verhältnis trans zu cis = 77 zu 23.

*Beispiel 3*

In zu Beispiel 1 oder 2 analoger Weise wurde durch Umsetzung von 1,4,4aα,9a-Tetrahydro-9aα-methyl-1α,4α-methanoanthrachinon mit

a) trans-Phytylchlorid, trans-Vitamin $K_1$ erhalten.

HPLC: Verhältnis trans zu cis = 98,3 zu 1,7.

b) mit trans/cis-Phytylchlorid, trans/cis-Vitamin-$K_1$.

HPLC: Verhältnis trans zu cis = 75 zu 25.

*Beispiel 4*

In einem Sulfierkolben versehen mit Rührer, Rückflusskühler und Argonbegasung wurden unter Argon 4,7 g (42 mMol) Kalium-tert.-butylat in 40 ml tert.-Butanol/Toluol (4 : 1) vorgelegt. Nach Abkühlen des Gemisches auf 0°C wurden 5,0 g (21 mMol) 1,4,-4aα,9a-Tetrahydro-9aα-methyl-1α,4α-methanoanthrachinon (hergestellt gemäss Beispiel 1) zugegeben. Die dunkelrote Mischung wurde anschliessend noch 10 Minuten bei 0°C nachgerührt. Hierauf wurden innert 15 Minuten 3,4 g (22,8 mMol) Dimethylallylbromid in 10 ml tert.-Butanol/Toluol (4 : 1) zugetropft und das Gemisch noch 30 Minuten bei 0°C gerührt. Dann wurden 20 ml Wasser zugegeben und das Gemisch am Rotationsverdampfer eingeengt. Der Rückstand wurde in 300 ml halbgesättigte NaCl-Lösung geleert, mit Hexan extrahiert, dann mit gesättigter NaCl-Lösung gewaschen, anschliessend über $Na_2SO_4$ getrocknet und dann eingeengt. Man erhielt 6,5 g 1,4,4a,9a-Tetrahydro-9aα-methyl-4aα-(3-methyl-2-butenyl)-1α,4α-methanoanthrachinon in Form von gelben Kristallen.

Zur Umkristallisation wurden diese Kristalle in 20 ml Äthanol gelöst, die Lösung zuerst auf 0°C abgekühlt und dann auf -20°C abgekühlt. Die ausgefallenen Kristalle wurden abfiltriert und mit eiskaltem Äthanol gewaschen. Anschliessend wurden sie während 1 Stunde bei 40°C am Wasserstrahlvakuum getrocknet. Man erhielt 4,4 g hellgelbe Kristalle mit einem Schmelzpunkt von 105-106°C.

4,4 g der vorhergehend erwähnten hellgelben Kristalle wurden in 20 ml Toluol gelöst und unter Argon während 15 Minuten am Rückfluss erhitzt. Anschliessend wurde das Gemisch abgekühlt und am Rotationsverdampfer eingeengt. Man erhielt 4,1 g eines gelben Öles, welches an einer 125 g $SiO_2$-Säule mit Hexan-Essigester (19 : 1) chromatographiert wurde. Auf diese Weise erhielt man 3,5 g Vitamin $K_{2(5)}$ als gelbes Öl. HPCL-Gehalt: 99%.

*Beispiel 5*

In zu Beispiel 4 analoger Weise wurden ausgehend von 1,4,4aα,9a-Tetrahydro-9aα-methyl-1α,4α-methanoanthrachinon folgende Verbindungen hergestellt:

durch Umsetzung mit Geranylbromid das 4aα-[(E)-3,7-Dimethyl-2,6-octadienyl]-1,4,4a,9a-tetrahydro-9aα-methyl-1α,4α-anthrachinon mit einem Schmelzpunkt von 68-69°C, und hieraus das Vitamin $K_{2(10)}$ als gelbe Kristalle mit einem Schmelzpunkt von 55-56°C; HPLC-Gehalt: 99,4%,

durch Umsetzung mit Farnesylbromid das 1,4,4a,-9a-Tetrahydro-9aα-methyl-4aα-[(all-E)-3,7,11-trimethyl-2,6,10-dodecatrienyl]-1α,4α-anthrachinon, und hieraus das Vitamin $K_{2(15)}$ als gelbes Öl; HPLC-Gehalt: 97,5%,

durch Umsetzung mit Geranylgeranylbromid das 1,4,4a,9a-Tetrahydro-9aα-methyl-4aα-[(all-E)-3,7,-11,15-tetramethyl-2,6,10,14-hexadecatetraenyl]-1α,4α-methanoanthrachinon, und hieraus das Vitamin $K_{2(20)}$ in Form von gelben Kristallen mit einem Schmelzpunkt von 37°C; HPLC-Gehalt: 96,3%,

durch Umsetzung mit Geranylfarnesylbromid das 1,4,4a,9a-Tetrahydro-9aα-methyl-4aα-[(all-E)-3,7,-11,15,19-pentamethyl-2,6,10,14,18-eicosapentaenyl]-1α,4α-methanoanthrachinon als gelbes Öl, und hieraus Vitamin $K_{2(25)}$ als gelbe Kristalle mit einem Schmelzpunkt von 43,5°C; HPLC-Gehalt: 99,7%.

*Beispiel 6*

In einem Sulfierkolben versehen mit Rührer, Rückflusskühler und Argonbegasung wurden unter Argon 1,3 g (11,6 mMol) Kalium-tert.-Butylat in 10 ml tert.-Butanol/Toluol (4 : 1) vorgelegt. Hierauf wurde das Gemisch auf 0°C abgekühlt und mit 1,2 g (4,84 mMol) 1,4,4a,8aα-Tetrahydro-6,7-dimethoxy-4aα-methyl-α,4α-methanonaphathalin-5,8-dion in 5 ml tert.-Butanol/Toluol (4 : 1) versetzt. Zu der erhaltenen roten Lösung wurden dann 2,3 g (6,3 mMol) trans-Phytylbromid in 5 ml tert.-Butanol/Toluol (4 : 1) innerhalb 30 Minuten bei 0°C zugetropft. Anschliessend wuden dem Gemisch noch 10 ml Wasser zugegeben und das Ganze am Rotationsverdampfer eingedampft. Der Rückstand wurde einmal mit 200 ml Hexan extrahiert, der Extrakt einmal mit Wasser gewaschen, dann über Natriumsulfat getrocknet, abfiltriert und eingeengt. Man erhielt 2,1 g eines braunes Öles, welches auf einer 70 g $SiO_2$-Säule mit Hexan-Essigester (4 : 1) chromatographiert wurde. So erhielt man 600 mg 1,4,4a,8a-Tetrahydro-6,7-dimethoxy-4aα-methyl-8aα-[(E)-3,7,11,15-tetramethyl-2-hexadecenyl]-1α,4α-methanonaphthalin-5,8-dion.

440 mg (0,84 mMol) des vorhergehend erhaltenen Öles wurden in 3 ml Toluol gelöst und unter Argon während 15 Minuten am Rückfluss erhitzt. Dann wurde das Gemisch abgekühlt und am Rotationsverdampfer eingeengt. Man erhielt 390 mg eines roten Öles. Dieses wurde auf einer 15 g $SiO_2$-Säule mit Hexan-Essigester (4 : 1) chromatographiert. Dabei wurden 340 mg Phylloubichinon als rotes Öl erhalten. HPLC-Gehalt: 89%.

Das als Ausgangsmaterial verwendete 1,4,4a,8aα-Tetrahydro-6,7-dimethoxy-4aα-methyl-1α-4α-me-

thanonaphthalin-5,8-dion wurde wie folgt hergestellt:

In einem Kolben wurden 1,0 g (5,49 mMol) 2,3-Dimethoxy-5-methyl-benzochinon in 4 ml Essigsäure vorgelegt. Dann wurden 1,4 ml (16,5 mMol) 1,3-Cyclopentadien zugesetzt und das Gemisch bei Raumtemperatur gerührt. Hierauf wurde die Lösung bei 40°C am Rotationsverdampfer eingeengt, der Rückstand zweimal mit je 200 ml Äther extrahiert, der Ätherextrakt dreimal mit Wasser und einmal mit gesättigter Natriumbicarbonatlösung gewaschen, dann über $Na_2SO_4$ getrocknet, filtriert und eingeengt. Man erhielt 1,5 g 4aα,5,8,8a-Tetrahydro-8aα-methyl-2,3-dimethoxy-5α,8α-methanonaphthochinon in Form eines orangefarbenen Öles. Dieses wurde auf einer 45 g $SiO_2$-Säule mit Hexan-Essigester (2 : 1) chromatographiert. Dies ergab 1,23 g reines 1,4,4a,8aα-Tetrahydro-6,7-dimethoxy-4aα-methyl-1α,4α-methanonaphthalin-5,8-dion.

*Beispiel 7*

In zu Beispiel 6 analoger Weise wurden ausgehend von 1,4,4a,8aα-Tetrahydro-6,7-dimethoxy-4aα-methyl-1α,4α-methanonaphthalin-5,8-dion folgende Verbindungen hergestellt:

durch Umsetzung mit Geranylbromid das 4aα-[(E)-3,7-Dimethyl-2,6-octadienyl]-1,4,4a,8a-tetrahydro-6,7-dimethoxy-8aα-methyl-1α,4α-methanonaphthalindion und hieraus das 2,3-Dimethoxy-5-methyl-6-[3,7-dimethyloctadien-(2,6)-yl]-benzochinon (1,4). HPLC-Reinheit: 100% trans.

durch Umsetzung mit Geranylgeranylbromid das 1,4,4a,8a-Tetrahydro-6,7-dimethoxy-4aα-methyl-8aα-[(all-E)-3,7,11,15-tetramethyl-2,6,10,14-hexadecatetraenyl]-1α,4α-methanonaphthalin-5,8-dion und hieraus das 2,3-Dimethoxy-5-methyl-6-[3,7,11,15-tetramethyl-hexadecatetraen-(2,6,10,-14)-yl-(1)]-benzochinon (1,4). HPLC-Reinheit: 100% all-trans.

durch Umsetzung mit Geranylfarnesylbromid das 1,4,4a,8a-Tetrahydro-6,7-dimethoxy-4aα-methyl-8aα-[(all-E)-3,7,11,15,19-pentamethyl-2,6,10,14,-18-eicosapentaenyl]-1α,4α-methanonaphathalin-5,8-dion und hieraus das 2,3-Dimethoxy-5-methyl-6-[3,7,11,15,19-pentamethyl-eicosapentaen-(2,-6,10,14,18)-yl-(1)]-benzochinon (1,4). HPLC-Reinheit: 95,6% all-trans.

durch Umsetzung mit Solanesylbromid das 1,4,-4a,8a-Tetrahydro-6,7-dimethoxy-8aα-methyl-4aα-[(all-E)-3,7,11,15,19,23,27,31,35-nonamethyl-2, 6,10,14,18,22,26,30,34-hexatriacontanonaenyl]-1α,4α-methanonaphthalin-5,8-dion und hieraus das 2,3-Dimethoxy-5-methyl-6-[3,7,11,15,19,23,27,-31,35-nonamethyl-hexatrikontanonaen-(2,6,10,-14,18,22,26,30,34)-yl-(1)]-benzochinon (1,4). HPLC-Reinheit: 98,7% all-trans.

**Patentansprüche für die Vertragsstaaten:**
BE, CH, DE, FR, GB, IT, LI, NL

1. Verfahren zur Herstellung von Chinonderivaten der allgemeinen Formel

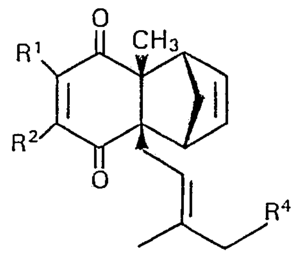

IV

worin $R^1$ und $R^2$ Methoxygruppen oder zusammen die Gruppe -CH = CH-CH = CH- darstellen und $R^4$ den 3,7,11-Trimethyldodecanrest oder einen Rest der Formel

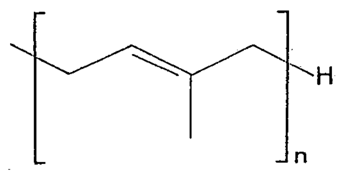

III

darstellt, worin n eine Zahl 0-12 bedeutet, dadurch gekennzeichnet, dass man eine Verbindung der allgemeinen Formel

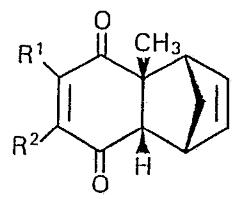

I

worin $R^1$ und $R^2$ die obige Bedeutung haben, mit einer Verbindung der allgemeinen Formel

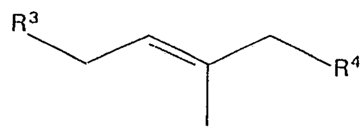

II

worin $R^3$ eine Abgangsgruppe bedeutet und $R^4$ die obige Bedeutung hat, umsetzt.

2. Verfahren zur Herstellung von Chinonderivaten der allgemeinen Formel

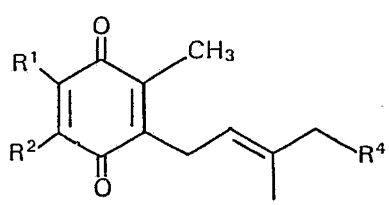

V

worin $R^1$ und $R^2$ Methoxygruppen oder zusammen die Gruppe -CH = CH-CH = CH- darstellen und $R^4$ den 3,7,11-Trimethyldodecanrest oder einen Rest der Formel

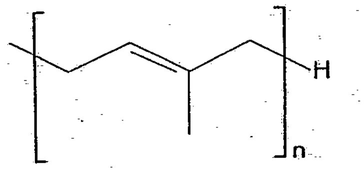

III

darstellt, worin n eine Zahl 0-12 bedeutet, dadurch gekennzeichnet, dass man eine Verbindung der allgemeinen Formel

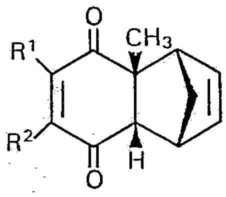

I

worin $R^1$ und $R^2$ die obige Bedeutung haben, mit einer Verbindung der allgemeinen Formel

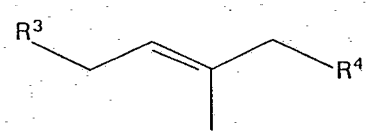

II

worin $R^3$ eine Abgangsgruppe darstellt und $R^4$ die obige Bedeutung hat,
umsetzt und die so erhaltene Verbindung der allgemeinen Formel

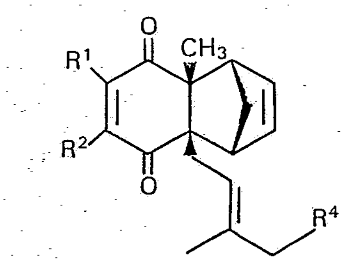

IV

worin $R^1$, $R^2$ und $R^4$ die obige Bedeutung haben, in die Verbindung der Formel V überführt.

3. Verfahren gemäss Anspruch 1 oder 2, dadurch gekennzeichnet, dass man die Umsetzung einer Verbindung der Formel I mit einer Verbindung der Formel II in einem inerten organischen Lösungsmittel und in Gegenwart einer starken Base durchführt.

4. Verfahren gemäss Anspruch 3, dadurch gekennzeichnet, dass man als inertes organischen Lösungsmittel ein apolares aprotisches oder ein polares protisches Lösungsmittel verwendet.

5. Verfahren nach Anspruch 3, dadurch gekennzeichnet, dass man als starke Base ein Alkalimetallamid, ein Lithiumdialkylamid oder ein Alkalimetall-tert.-Butylat verwendet.

6. Verfahren gemäss einem der Ansprüche 1-5, dadurch gekennzeichnet, dass man die Umsetzung einer Verbindung der Formel I mit einer Verbindung der Formel II bei einer Temperatur von etwa −20°C bis etwa +30°C, vorzugsweise bei etwa −5°C bis etwa +10°C und insbesondere bei etwa 0°C bis etwa +5°C durchführt.

7. Verbindungen der allgemeinen Formel

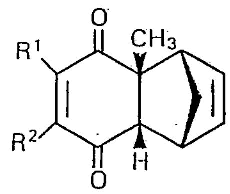

I

worin $R^1$ und $R^2$ Methoxygruppen oder zusammen die Gruppe -CH=CH-CH=CH- darstellen.

8. Verbindungen der allgemeinen Formel

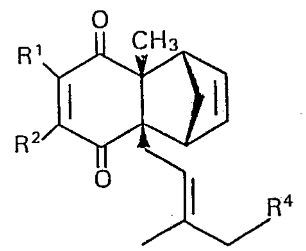

IV

worin $R^1$ und $R^2$ Methoxygruppen oder zusammen die Gruppe -CH=CH-CH=CH- darstellen, $R^4$ den 3,7,11-Trimethyldodecanrest oder einen Rest der Formel

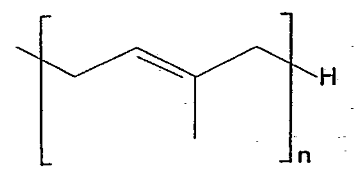

III

und n eine Zahl 0-12 bedeuten.

**Patentansprüche für den Vertragsstaat:** AT

1. Verfahren zur Herstellung von Chinonderivaten der allgemeinen Formel

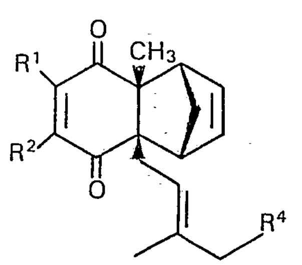

IV

worin $R^1$ und $R^2$ Methoxygruppen oder zusammen die Gruppe -CH=CH-CH=CH- darstellen und $R^4$ den 3,7,11-Trimethyldodecanrest oder einen Rest der Formel

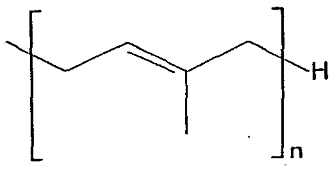

III

darstellt, worin n eine Zahl 0-12 bedeutet, dadurch gekennzeichnet, dass man eine Verbindung der allgemeinen Formel

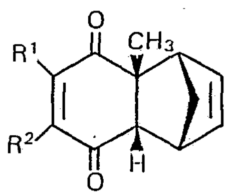

I

worin $R^1$ und $R^2$ die obige Bedeutung haben, mit einer Verbindung der allgemeinen Formel

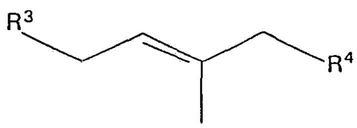

II

worin $R^3$ eine Abgangsgruppe bedeutet und $R^4$ die obige Bedeutung hat,
umsetzt.

2. Verfahren zur Herstellung von Chinonderivaten der allgemeinen Formel

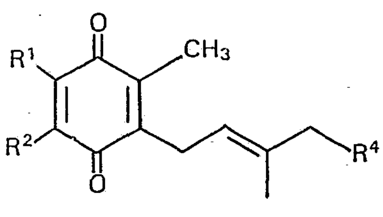

V

worin $R^1$ und $R^2$ Methoxygruppen oder zusammen die Gruppe -CH = CH-CH = CH- darstellen und $R^4$ den 3,7,11-Trimethyldodecanrest oder einen Rest der Formel

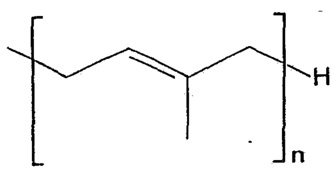

III

darstellt, worin n eine Zahl 0-12 bedeutet, dadurch gekennzeichnet, dass man eine Verbindung der allgemeinen Formel

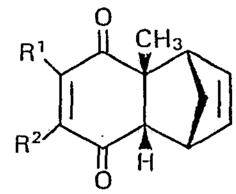

I

worin $R^1$ und $R^2$ die obige Bedeutung haben, mit einer Verbindung der allgemeinen Formel

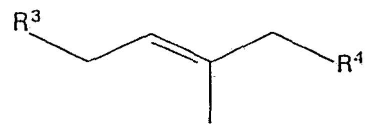

II

worin $R^3$ eine Abgangsgruppe darstellt und $R^4$ die obige Bedeutung hat,
umsetzt und die so erhaltene Verbindung der allgemeinen Formel

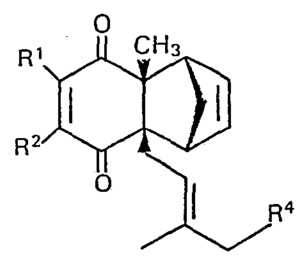

IV

worin $R^1$, $R^2$ und $R^4$ die obige Bedeutung haben, in die Verbindung der Formel V überführt.

3. Verfahren gemäss Anspruch 1 oder 2, dadurch gekennzeichnet, dass man die Umsetzung einer Verbindung der Formel I mit einer Verbindung der Formel II in einem inerten organischen Lösungsmittel und in Gegenwart einer starken Base durchführt.

4. Verfahren gemäss Anspruch 3, dadurch gekennzeichnet, dass man als inertes organischen Lösungsmittel ein apolares aprotisches oder ein polares protisches Lösungsmittel verwendet.

5. Verfahren nach Anspruch 3, dadurch gekennzeichnet, dass man als starke Base ein Alkalimetallamid, ein Lithiumdialkylamid oder ein Alkalimetall-tert.-Butylat verwendet.

6. Verfahren gemäss einem der Ansprüche 1-5, dadurch gekennzeichnet, dass man die Umsetzung einer Verbindung der Formel I mit einer Verbindung der Formel II bei einer Temperatur von etwa -20°C bis etwa +30°C, vorzugsweise bei etwa -5°C bis etwa +10°C und insbesondere bei etwa 0°C bis etwa +5°C durchführt.

**Claims for the Contracting States:**
BE, CH, DE, FR, GB, IT, LI, NL

1. A process for the manufacture of quinone derivatives of the general formula

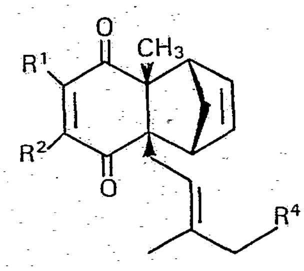

IV

wherein $R^1$ and $R^2$ represent methoxy groups or together represent the group -CH=CH-CH=CH- and $R^4$ represents the 3,7,11-trimethyldodecane residue or a residue of the formula

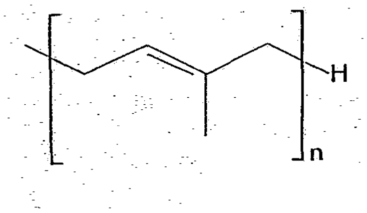

III

wherein n signifies a number 0-12, characterized by reacting a compound of the general formula

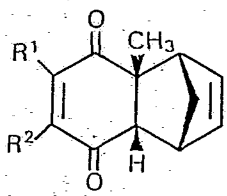

I

wherein $R^1$ and $R^2$ have the above significance, with a compound of the general formula

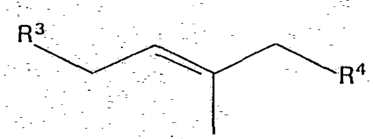

II

wherein $R^3$ signifies a leaving group and $R^4$ has the above significance.

2. A process for the manufacture of quinone derivatives of the general formula

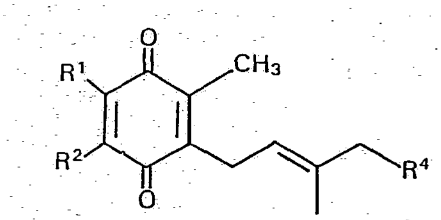

V

wherein $R^1$ and $R^2$ represent methoxy groups or together represent the group -CH=CH-CH=CH- and $R^4$ represents the 3,7,11-trimethyldodecane residue or a residue of the formula

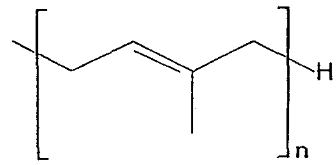

III

wherein n signifies a number 0-12, characterized by reacting a compound of the general formula

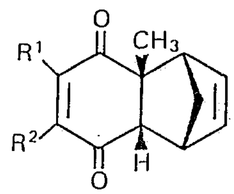

I

wherein $R^1$ and $R^2$ have the above significance with a compound of the general formula

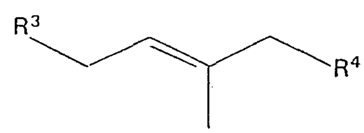

II

wherein $R^3$ represents a leaving group and $R^4$ has the above significance, and converting the thus-obtained compound of the general formula

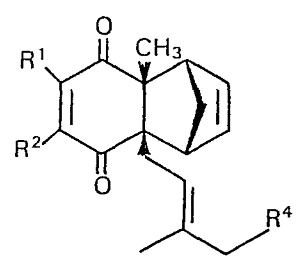

IV

wherein $R^1$, $R^2$ and $R^4$ have the above significance, into the compound of formula V.

3. A process in accordance with claim 1 or 2, characterized in that the reaction of a compound of formula I with a compound of formula II is carried out in an inert organic solvent and in the presence of a strong base.

4. A process in accordance with claim 3, characterized in that an apolar aprotic solvent or a polar protic solvent is used as the inert organic solvent.

5. A process according to claim 3, characterized in that an alkali metal amide, a lithium dialkylamide or an alkali metal tert.-butylate is used as the strong base.

6. A process in accordance with any one of claims 1-5, characterized in that the reaction of a compound of formula I with a compound of formula II is carried out at a temperature of about −20°C to about

+30°C, preferably at about −5°C to about +10°C and especially at about 0°C to about +5°C.

7. Compounds of the general formula

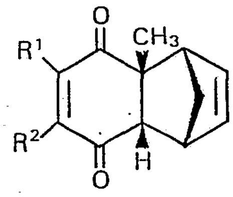

I

wherein $R^1$ and $R^2$ represent methoxy groups or together represent the group -CH=CH-CH=CH-.

8. Compounds of the general formula

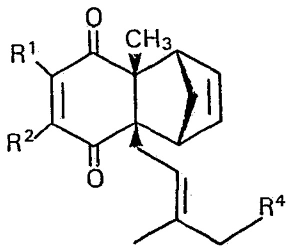

IV

wherein $R^1$ and $R^2$ represent methoxy groups or together represent the group -CH=CH-CH=CH-, $R^4$ signifies the 3,7,11-trimethyldodecane residue or a residue of the formula

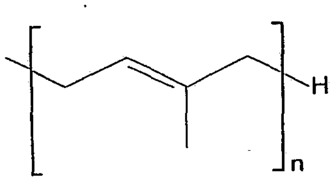

III

and n signifies a number 0-12.

**Claims for the Contracting Sate:** AT

1. A process for the manufacture of quinone derivatives of the general formula

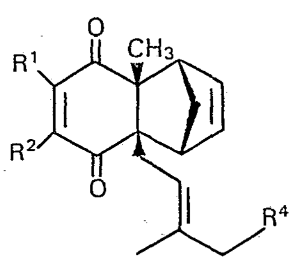

IV

wherein $R^1$ and $R^2$ represent methoxy groups or together represent the group -CH=CH-CH=CH- and $R^4$ represents the 3,7,11-trimethyldodecane residue or a residue of the formula

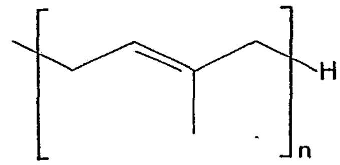

III

wherein n signifies a number 0-12, characterized by reacting a compound of the general formula

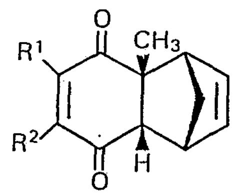

I

wherein $R^1$ and $R^2$ have the above significance, with a compound of the general formula

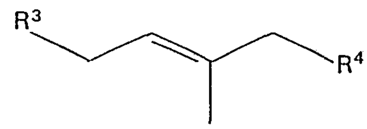

II

wherein $R^3$ signifies a leaving group and $R^4$ has the above significance.

2. A process for the manufacture of quinone derivatives of the general formula

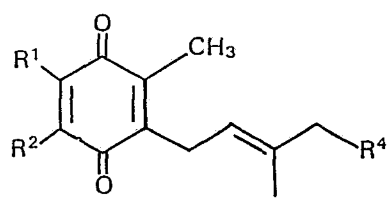

V

wherein $R^1$ and $R^2$ represent methoxy groups or together represent the group -CH=CH-CH=CH- and $R^4$ represents the 3,7,11-trimethyldodecane residue or a residue of the formula

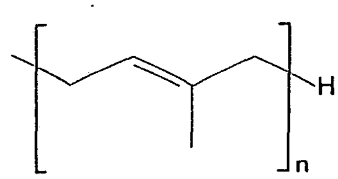

III

wherein n signifies a number 0-12, characterized by reacting a compound of the general formula

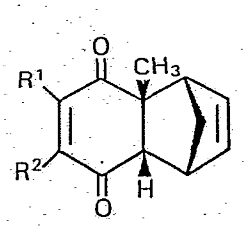

I

wherein $R^1$ and $R^2$ have the above significance with a compound of the general formula

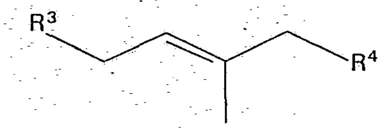

II

wherein $R^3$ represents a leaving group and $R^4$ has the above significance,
and converting the thus-obtained compound of the general formula

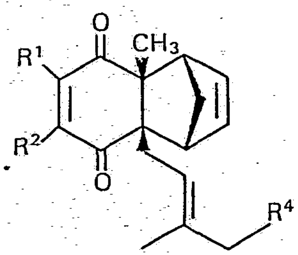

IV

wherein $R^1$, $R^2$ and $R^4$ have the above significance,
into the compound of formula V.

3. A process in accordance with claim 1 or 2, characterized in that the reaction of a compound of formula I with a compound of formula II is carried out in an inert organic solvent and in the presence of a strong base.

4. A process in accordance with claim 3, characterized in that an apolar aprotic solvent or a polar protic solvent is used as the inert organic solvent.

5. A process according to claim 3, characterized in that an alkali metal amide, a lithium dialkylamide or an alkali metal tert.-butylate is used as the strong base.

6. A process in accordance with any one of claims 1-5, characterized in that the reaction of a compound of formula I with a compound of formula II is carried out at a temperature of about −20°C to about +30°C, preferably at about −5°C to about +10°C and especially at about 0°C to about +5°C.

**Revendications pour les Etats contractants:**
BE, CH, DE, FR, GB, IT, LI, NL

1. Procédé pour la préparation de dérivés quinoniques de formule générale

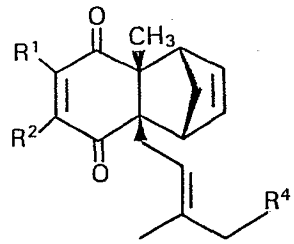

IV

dans laquelle $R^1$ et $R^2$ représentent des groupes méthoxy ou forment ensemble le groupe -CH=CH-CH=CH- et $R^4$ représente le reste du 3,7,11-triméthyldodécane ou un reste de formule

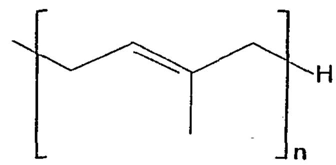

III

dans laquelle n est un nombre de 0 à 12, charactérisé en ce que l'on fait réagir un composé de formule générale

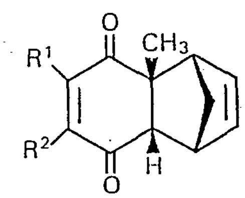

I

dans laquelle $R^1$ et $R^2$ ont les significations indiquées ci-dessus,
avec un composé de formule générale

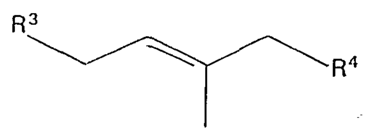

II

dans laquelle $R^3$ représente un groupe éliminable et $R^4$ a les significations indiquées ci-dessus.

2. Procédé de préparation de dérivés quinoniques de formule générale

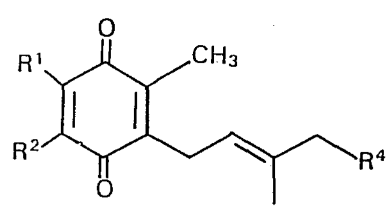

V

dans laquelle $R^1$ et $R^2$ représentent des groupes méthoxy ou forment ensemble le groupe -CH=CH-CH=CH- et $R^4$ représente le reste du 3,7,11-triméthyldodécane ou un reste de formule

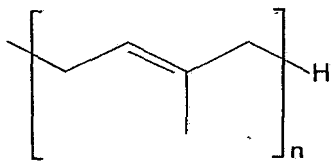

III

dans laquelle n est un nombre allant de 0 à 12, caractérisé en ce que l'on fait réagir un composé de formule générale

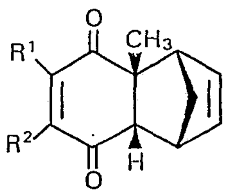

I

dans laquelle $R^1$ et $R^2$ ont les significations indiquées ci-dessus,
avec un composé de formule générale

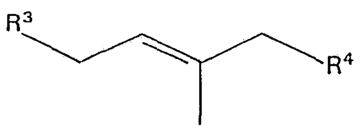

II

dans laquelle $R^3$ représente un groupe éliminable et $R^4$ a les significations indiquées ci-dessus,
ce qui donne un composé de formule générale

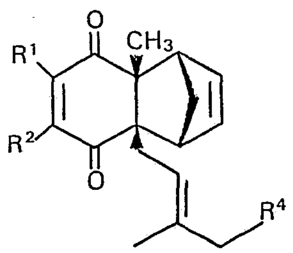

IV

dans laquelle $R^1$, $R^2$ et $R^4$ ont les significations indiquées ci-dessus,
qu'on convertit en le composé de formule V.

3. Procédé selon la revendication 1 ou 2, caractérisé en ce que la réaction d'un composé de formule I avec un composé de formule II est effectuée dans un solvant organique inerte et en présence d'une base forte.

4. Procédé selon la revendication 3, caractérisé en ce que l'on utilise en tant que solvant organique inerte un solvant aprotonique apolaire ou un solvant protonique polaire.

5. Procédé selon la revendication 3, caractérisé en ce que l'on utilise en tant que base forte un amidure de métal alcalin, un dialkylamidure de lithium ou un tert.-butylate de métal alcalin.

6. Procédé selon l'une des revendications 1 à 5, caractérisé en ce que la réaction d'un composé de formule I avec un composé de formule II est effectuée à une température allant de −20 à +30°C environ, de préférence de −5 à +10°C environ et plus spécialement de 0 à +5°C environ.

7. Composés de formule générale

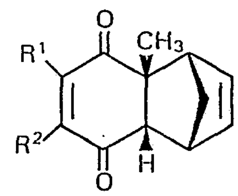

I

dans laquelle $R^1$ et $R^2$ représentent des groupes méthoxy ou forment ensemble le groupe -CH=CH-CH=CH-.

8. Composés de formule générale

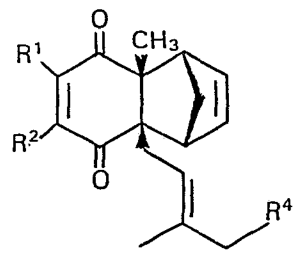

IV

dans laquelle $R^1$ et $R^2$ représentent des groupes méthoxy ou forment ensemble le groupe -CH=CH-CH=CH- et $R^4$ représente le reste du 3,7,11-triméthyldodécane ou un reste de formule

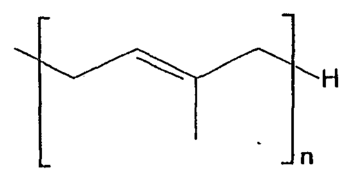

III

et n est un nombre allant de 0 à 12.

**Revendications pour l'Etat contractant:** AT

1. Procédé pour la préparation de dérivés quinoniques de formule générale

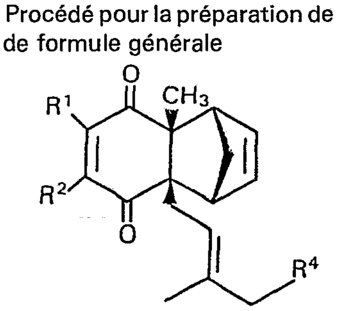

IV

dans laquelle $R^1$ et $R^2$ représentent des groupes méthoxy ou forment ensemble le groupe -CH=CH-CH=CH- et $R^4$ représente le reste du 3,7,11-triméthyldodécane ou un reste de formule

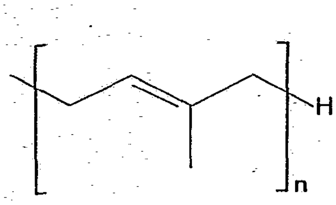

III

dans laquelle n est un nombre allant de 0 à 12, charactérisé en ce que l'on fait réagir un composé de formule générale

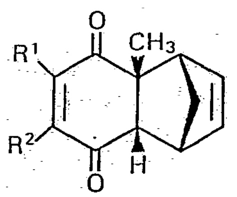

I

dans laquelle $R^1$ et $R^2$ ont les significations indiquées ci-dessus,
avec un composé de formule générale

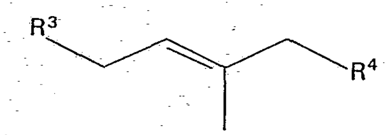

II

dans laquelle $R^3$ représente un groupe éliminable et $R^4$ a les significations indiquées ci-dessus.

2. Procédé de préparation de dérivés quinoniques de formule générale

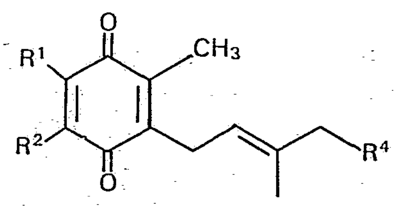

V

dans laquelle $R^1$ et $R^2$ représentent des groupes méthoxy ou forment ensemble le groupe -CH=CH-CH=CH- et $R^4$ représente le reste du 3,7,11-triméthyldodécane ou un reste de formule

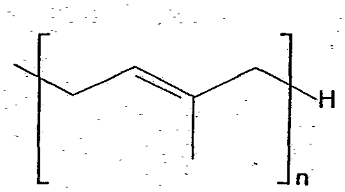

III

dans laquelle n est un nombre allant de 0 à 12, caractérisé en ce que l'on fait réagir un composé de formule générale

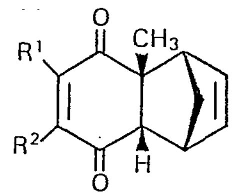

I

dans laquelle $R^1$ et $R^2$ ont les significations indiquées ci-dessus,
avec un composé de formule générale

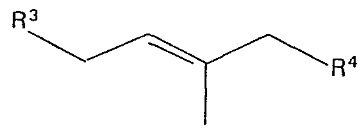

II

dans laquelle $R^3$ représente un groupe éliminable et $R^4$ a les significations indiquées ci-dessus,
ce qui donne un composé de formule générale

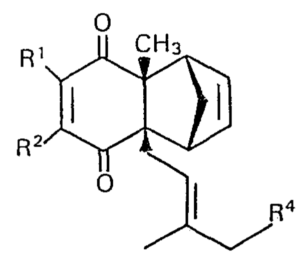

IV

dans laquelle $R^1$, $R^2$ et $R^4$ ont les significations indiquées ci-dessus,
qu'on convertit en le composé de formule V.

3. Procédé selon la revendication 1 ou 2, caractérisé en ce que la réaction d'un composé de formule I avec un composé de formule II est effectuée dans un solvant organique inerte et en présence d'une base forte.

4. Procédé selon la revendication 3, caractérisé en ce que l'on utilise en tant que solvant organique inerte un solvant aprotonique apolaire ou un solvant protonique polaire.

5. Procédé selon la revendication 3, caractérisé en ce que l'on utilise en tant que base forte un amidure de métal alcalin, un dialkylamidure de lithium ou un tert.-butylate de métal alcalin.

6. Procédé selon l'une des revendications 1 à 5, caractérisé en ce que la réaction d'un composé de formule I avec un composé de formule II est effectuée à une température allant de −20 à +30°C environ, de préférence de −5 à +10°C environ et plus spécialement de 0 à +5°C environ.